# EUROPÄISCHE PATENTSCHRIFT

(11) **EP 1 698 301 B1**
(45) Veröffentlichungstag und Bekanntmachung des Hinweises auf die Patenterteilung: **12.10.2011**
(21) Anmeldenummer: 06110682.9
(22) Anmeldetag: 06.03.2006
(51) Int. Cl.: A61C 17/20, H01L 41/107

(54) **Zahnärztliche Vorrichtung zur Behandlung mittels Ultraschall**
Dental device for ultrasound treatment
Dispositif dentaire de traitement par ultrasons

(30) Priorität: 04.03.2005 DE 102005010556
(43) Veröffentlichungstag der Anmeldung: 06.09.2006
(73) Patentinhaber: Sirona Dental Systems GmbH, 64625 Bensheim (DE)
(72) Erfinder: Rein, Matthias, 64653, Lorsch (DE)
(74) Vertreter: Sommer, Peter

(56) Entgegenhaltungen:
- EP-A- 0 914 809
- US-A- 4 012 647
- US-A- 4 038 571
- US-A- 5 309 590
- US-A- 6 139 320
- US-A1- 2002 138 090

## Beschreibung

### Technisches Gebiet

Die vorliegende Erfindung betrifft eine zahnärztliche Vorrichtung zur Behandlung mittels Ultraschall mit einem Transformator zur Umwandlung einer elektrischen Eingangsspannung in eine für höhere Leistungen erforderliche höhere elektrische Ausgangsspannung sowie ein Instrument, das einen Wandler zur Umwandlung der elektrischen Ausgangsspannung in eine mechanische Ultraschallschwingung aufweist.

### Stand der Technik

Aus dem Stand der Technik sind unterschiedliche zahnärztliche Ultraschallgeräte bekannt, die beispielsweise der Entfernung von Zahnstein dienen. Die bekannten Vorrichtungen umfassen regelmäßig die folgenden Komponenten.

Zum einen ist ein Transformator vorgesehen. Der Transformator dient der Umwandlung einer elektrischen Eingangsspannung in eine höhere elektrische Ausgangsspannung. Dies ist erforderlich, um eine höhere Abtragsleistung des Zahnsteinentferners o. Ä. zu erzielen. Die in herkömmlichen Geräten verwendeten Transformatoren beruhen auf dem elektromagnetischen Prinzip und bedürfen daher eines großen Aufbaus. Demzufolge sind die bekannten Transformatoren groß bzw. voluminös.

Zum anderen weisen die bekannten Vorrichtungen ein Instrument auf, mit dem der Zahnarzt die zu behandelnden Stellen an den Zähnen bearbeitet. In das Handgerät ist ein Wandler integriert. Dieser Wandler dient der Umwandlung der elektrischen Ausgangsspannung in eine mechanische Ultraschallschwingung eines entsprechenden Werkzeuges. Derartige Wandler arbeiten herkömmlicherweise nach dem magnetostriktiven Prinzip oder dem piezoelektrischen Prinzip, wobei die magnetostriktiven Wandler eine geringere elektrische Ausgangsspannung benötigen als die piezoelektrischen Wandler.

Darüber hinaus umfassen die bekannten Vorrichtungen entsprechende Steuerungseinrichtungen zur Steuerung der Vorrichtung. Die bekannten Vorrichtungen sind dabei derart aufgebaut, dass der Transformator und die Steuerungseinrichtung in einem separaten Gerät untergebracht sind, um über ein Kabel bzw. eine Leitung mittelbar mit dem Instrument und dem darin angeordneten Wandler verbunden zu sein. Über das Kabel bzw. die Leitung erfolgt sowohl die Energieversorgung als auch die Übermittlung der Steuerbefehle von der Steuerungseinrichtung. Das die Steuerungseinrichtung und den Transformator aufnehmende Gerät muss im Behandlungsumfeld angeordnet werden und wird von der Bedienungsperson, wie beispielsweise dem Zahnarzt oder der Zahnarzthelferin, als störend empfunden, zumal dieses sehr voluminös ist, was die Handhabung nachhaltig erschwert.

Dokument US 20020138090 offenbart ein Ultraschallskalpell gemäß dem Oberbegriff des Anspruchs 1.

Der vorliegenden Erfindung liegt somit die Aufgabe zugrunde, eine zahnärztliche Vorrichtung zur Behandlung mittels Ultraschall zu schaffen, die einen kompakten Aufbau bei gleichzeitig hoher Leistung hat und deren Handhabung vereinfacht ist.

### Darstellung der Erfindung

Die Lösung der vorgenannten Aufgabe erfolgt anhand der Merkmale des Patentanspruches 1. Vorteilhafte Ausführungsformen der Erfindung sind Gegenstand der Unteransprüche.

Die erfindungsgemäße Vorrichtung ist eine zahnärztliche Vorrichtung zur Behandlung mittels Ultraschall. Es kann sich beispielsweise um eine Vorrichtung zum Entfernen von Zahnstein o. Ä. handeln. Die Vorrichtung weist einen Transformator auf. Der Transformator dient der Umwandlung einer elektrischen Eingangsspannung in eine für höhere Leistungen erforderliche höhere elektrische Ausgangsspannung. Als Transformator kommen beispielsweise herkömmliche, auf dem elektromagnetischen Prinzip beruhende Transformatoren in Frage.

Die erfindungsgemäße Vorrichtung weist ferner ein Instrument auf, mit dem die Bearbeitung der zu behandelnden Stelle im Mund des Patienten erfolgt. Das Instrument ist mit einem Wandler versehen, der der Umwandlung der elektrischen Ausgangsspannung in eine mechanische Ultraschallschwingung dient. Als Wandler kommen beispielsweise magnetostriktive Wandler in Betracht, die aus dem Stand der Technik bekannt sind.

Um einen kompakteren Aufbau der erfindungsgemäßen Vorrichtung zu erzielen, ist der Transformator unmittelbar an dem Instrument angeordnet. Unter Unmittelbarkeit ist hierbei u.a. zu verstehen, dass der Transformator nicht anderenorts aufgestellt ist, um mit dem Instrument über ein Kabel o. Ä. verbunden werden zu müssen. So kann der Transformator beispielsweise in das Instrument integriert sein. Auf diese Weise wird zwar das Instrument gegebenenfalls größer, jedoch ist dies weniger behindernd als ein großes externes Gerät, so dass eine leichtere Handhabbarkeit der gesamten Vorrichtung erzielt ist.

Erfindungsgemäß ist der Transformator ein piezoelektrischer Transformator. Ein derartiger piezoelektrischer Transformator besteht beispielsweise aus zwei Piezoelementen, wobei die elektrische Energie einenendes in mechanische Energie und anderenendes wieder in elektrische Energie gewandelt wird, wodurch die niedrige elektrische Eingangsspannung in eine erhöhte elektrische Ausgangsspannung umgewandelt werden kann. Auch kann dadurch ein hochfrequenterer Strom erzeugt werden. Der piezoelektrische Transformator hat den Vorteil, dass er im Vergleich zu den herkömmlichen gewickelten Transformatoren ein geringeres Gewicht und ein geringeres Volumen hat. Somit wird weder das Gewicht noch das Volumen des Instruments trotz der Einbindung des Transformators merklich vergrößert, was zu einer weiteren Verbesserung der Handhabung der erfindungsgemäßen Vorrichtung führt.

Erfindungsgemäß ist der piezoelektrische Transformator an einer durch den Wandler in Schwingung versetzten Resonanz-/Schwungmasse angeordnet. Dies ermöglicht eine besonders kleine Bauweise des Instrumentes, wodurch die Handhabung der Vorrichtung weiter vereinfacht werden kann.

In einer besonders bevorzugten Ausführungsform ist der piezoelektrische Transformator im Bereich eines Schwingungsbauchs der Resonanz-/Schwungmasse angeordnet. Die Resonanz-/Schwungmasse wird üblicherweise in einer Mode betrieben, die auch eine elastische Verformung der Resonanz-/Schwungmasse bewirkt. Dadurch entstehen entlang der Resonanz-/Schwungmasse mindestens ein Schwingungsknoten und zwei Schwingungsbäuche. Eine Anordnung in einem Schwingungsknoten würde den piezoelektrischen Transformator durch die in unterschiedliche Richtungen weisenden Beschleunigungen zur Erzeugung einer Wechselspannung mit der Schwingfrequenz der Resonanz-/Schwungmasse anregen. Einerseits würde dem schwingenden System dadurch Energie entzogen werden und andererseits würde eine Rückspeisung der hochfrequenten Wechselspannung entstehen, die herausgefiltert werden müsste.

In einer alternativen besonders bevorzugten Ausführungsform ist der piezoelektrische Transformator mechanisch entkoppelt an der Resonanz-/Schwungmasse angeordnet. Damit lassen sich dieselben Vorteile wie bei einer Anordnung in einem Schwingungsbauch erzielen.

In einer weiteren vorteilhaften Ausführungsform der erfindungsgemäßen zahnärztlichen Vorrichtung ist der Wandler ein piezoelektrischer Wandler. Ein piezoelektrischer Wandler kann im Gegensatz zu den herkömmlicherweise eingesetzten magnetostriktiven Wandlern besonders klein und leichtgewichtig aufgebaut sein, was zu einem geringen Gewicht und zu einer geringen Größe des Instrumentes führt, wodurch wiederum die Handhabbarkeit der Vorrichtung verbessert ist.

Um eine besonders kleine Bauweise des Instrumentes zu ermöglichen, umfassen der piezoelektrische Transformator und/oder der piezoelektrische Wandler in einer besonders bevorzugten Ausführungsform der Erfindung mehrere übereinander geschichtete, ebene Piezoelemente.

In einer weiteren vorteilhaften Ausführungsform der erfindungsgemäßen zahnärztlichen Vorrichtung sind die Piezoelemente Quarz-, Turmalin- oder Seignettesalz-Bauelemente oder Piezokeramiken wie vorzugsweise PZT. Die piezoelektrischen Materialien sind somit natürliche Kristalle, die besonders gut hierfür geeignet sind.

In einer weiteren bevorzugten Ausführungsform der erfindungsgemäßen Vorrichtung ist eine Steuerungseinrichtung, wie beispielsweise eine Steuerelektronik, zur Steuerung der Vorrichtung vorgesehen. Die Steuerungseinrichtung ist in das Instrument integriert. Die Steuerungseinrichtung kann neben der Elektronik auch entsprechende Bedienelemente umfassen, die fortan direkt an dem Instrument betätigt werden können. In jedem Fall kann die Größe des Beistellgerätes weiter verringert werden.

Vorteilhafterweise ist das Instrument von einem Gehäuse umfasst. In dem Gehäuse können alle Bauteile des Instruments untergebracht sein, die zur Erzeugung von Schwingenergie dienen.

Gemäß einer weiteren vorteilhaften Ausführungsform der Erfindung ist eine Energieversorgungseinrichtung vorgesehen, die in das Gehäuse des Instruments integriert ist. Hierbei kann es sich beispielsweise um Akkumulatoren o. Ä. handeln. Da bei dieser Ausführungsform sowohl der Transformator als auch die Energieversorgungseinrichtung in das Gehäuse des Instruments integriert sind, kann das Instrument auch drahtlos betrieben werden, da weder die Steuersignale noch die notwendige Energie über ein Kabel übertragen werden müssten.

In einer weiteren bevorzugten Ausführungsform der erfindungsgemäßen zahnärztlichen Vorrichtung weist das Instrument ein Werkzeug auf, das durch den Wandler in mechanische Ultraschallschwingungen versetzt werden kann. Bei diesem Werkzeug kann es sich beispielsweise um einen Zahnsteinmeißel handeln, der hakenähnlich ausgebildet ist.

In einer besonders bevorzugten Ausführungsform der erfindungsgemäßen zahnärztlichen Vorrichtung kann das an dem Instrument angeordnete Werkzeug ausgetauscht werden. Auf diese Weise muss bei einem Wechsel des Patienten lediglich das entsprechende Werkzeug ausgetauscht werden, um den bestehenden hygienischen Anforderungen zu genügen.

In einer bevorzugten Ausführungsform der zahnärztlichen Vorrichtung ist eine Lampe am Niederspannungsteil angeschlossen und in das Gehäuse integriert. Der Niederspannungsteil ist der Teil auf der Eingangsseite des Transformators. Die Spannung muss dann nicht erneut zum Betrieb der Lampe transformiert werden. Eine derartige Beleuchtung, die auf das Beahndlungsfeld gerichtet ist, sorgt für eine bessere Sicht.

Eine besonders bevorzugte Ausführungsform der erfindungsgemäßen zahnärztlichen Vorrichtung sieht eine Einrichtung zur Kühlung des Instruments vor, wobei die Kühleinrichtung so ausgelegt ist, dass der Transformator durch die Kühleinrichtung mitgekühlt wird. Eine solche Kühleinrichtung dient bei Ultraschallinstrumenten häufig zur Kühlung des Werkzeugs und kann beispielsweise durch die Zu- und Ableitung von Wasser realisiert werden. Ein Transformator erzeugt prinzipbedingt immer Wärmeenergie, die durch eine solche Kühleinrichtung besonders leicht abgeführt werden kann.

### Kurzbeschreibung der Zeichnung

Fig. 1 zeigt die schematische Darstellung einer ersten Ausführungsform der erfindungsgemäßen zahnärztlichen Vorrichtung zur Behandlung mittels Ultraschall und
Fig. 2 zeigt die schematische Darstellung einer zweiten Ausführungsform der erfindungsgemäßen zahnärztlichen Vorrichtung zur Behandlung mittels Ultraschall.

### Ausführungsbeispiele der Erfindung

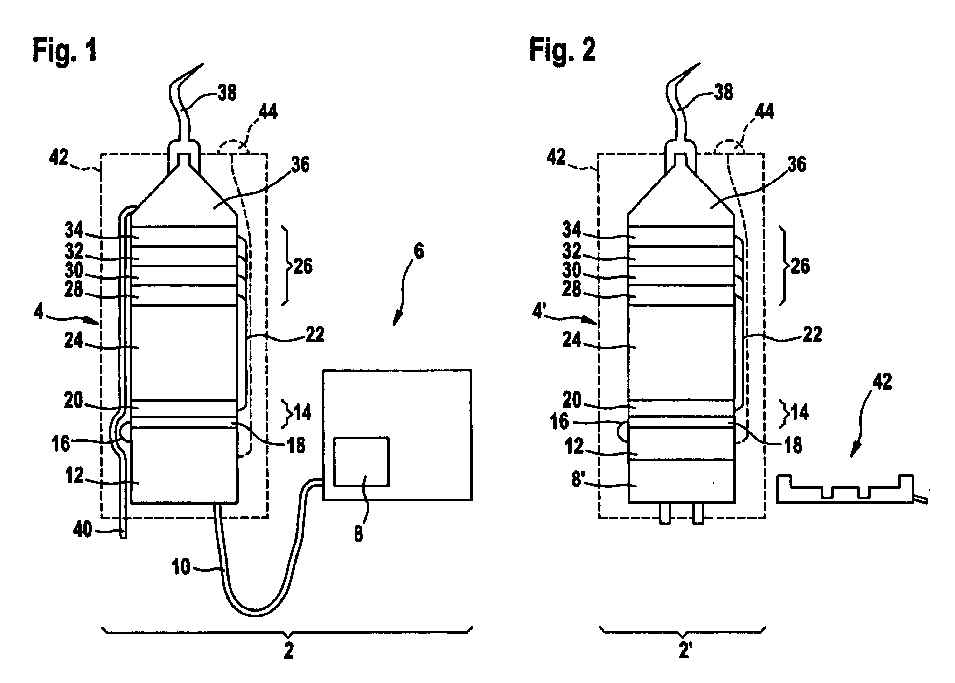

Fig. 1 zeigt die schematische Darstellung einer ersten Ausführungsform der erfindungsgemäßen zahnärztlichen Vorrichtung 2 zur Behandlung mittels Ultraschall.

In der ersten Ausführungsform der Erfindung lässt sich die Vorrichtung 2 im Wesentlichen in zwei Einheiten unterteilen, nämlich in ein Instrument 4 und ein peripheres Gerät 6. In dem peripheren Gerät 6 ist eine Energieversorgungseinrichtung 8 vorgesehen, die die zum Betrieb des Instrumentes 4 notwendige elektrische Energie zur Verfügung stellt. Das Instrument 4 ist über ein Kabel 10 mit dem peripheren Gerät 6 und somit mit der Energieversorgungseinrichtung 8 verbunden.

Das Instrument 4 weist eine Steuerungseinrichtung 12 zur Steuerung der Vorrichtung 2 auf. Indem die Steuerungseinrichtung 12 in das Instrument 4 integriert ist, wird die Handhabung der erfindungsgemäßen Vorrichtung 2 stark vereinfacht und die Größe des peripheren Gerätes 6 ist reduziert. Die Steuerungseinrichtung 12, die in der vorliegenden Ausführungsform von einer leistungsstarken Steuerelektronik gebildet wird, ist mit dem Kabel 10 verbunden.

Über das Kabel 10 und die Steuerungseinrichtung 12 versorgt die Energieversorgungseinrichtung 8 einen Transformator 14 mit einer elektrischen Eingangsspannung, was anhand der Leitung 16 angedeutet ist. Der Transformator 14 ist unmittelbar an dem Instrument 4 und nicht in dem peripheren Gerät 6 angeordnet. Bei dem Transformator 14 handelt es sich um einen piezoelektrischen Transformator 14. Der piezoelektrische Transformator 14 umfasst in der vorliegenden Ausführungsform zwei übereinander geschichtete, ebene Piezoelemente, nämlich ein eingangsseitiges Piezoelement 18 und ein ausgangsseitiges Piezoelement 20. Während an das eingangsseitige Piezoelement 18 - wie zuvor beschrieben - eine elektrische Eingangsspannung angelegt wird, wird an dem ausgangsseitigen Piezoelement 20 eine elektrische Ausgangsspannung abgegriffen, wie dies anhand der Leitung 22 angedeutet ist.

Die Steuerungseinrichtung 12 entkoppelt die Piezoelemente 18, 20, 28, 30, 32, 34 durch eine entsprechende Beschaltung elektrisch gegen das Versorgungsnetz, da die schwingenden Piezoelemente immer hochfrequente Spannungen erzeugen, die gegebenenfalls das Versorgungsnetz stören könnten.

Die Entkopplung könnte auch in der Energieversorgungseinrichtung 8 vorgesehen sein.

Indem das eingangsseitige Piezoelement 18 mit der elektrischen Eingangsspannung beaufschlagt wird, wird die elektrische Energie in eine mechanische Energie umgewandelt, nämlich in eine Schwingung des eingangsseitigen Piezoelementes 18, das sich abwechselnd ausdehnt und wieder zusammenzieht (Piezoeffekt). Diese mechanische Energie wirkt nunmehr auf das ausgangsseitige Piezoelement 20, das entsprechend zusammengedrückt wird bzw. sich wieder ausdehnt. Auf Grund dieser mechanischen Beanspruchung entstehen auf dem ausgangsseitigen Piezoelement 20 elektrische Ladungen (inverser Piezoeffekt), so dass an dem ausgangsseitigen Piezoelement 20 eine elektrische Ausgangsspannung abgegriffen werden kann.

Der piezoelektrische Transformator 14 bzw. dessen Piezoelemente 16, 18 sind so ausgelegt, dass die elektrische Ausgangsspannung höher als die elektrische Eingangsspannung ist. Bei den Piezoelementen 18, 20 handelt es sich um Quarz-Bauelemente. Alternativ ist der Einsatz von Piezokeramiken denkbar.

Der piezoelektrische Transformator 14 ist an einer Resonanz-/Schwungmasse 24 angeordnet, die beispielsweise aus Stahl besteht und durch einen nachfolgend beschriebenen Wandler 26 in Schwingung versetzt wird. Die Masse der Resonanz-/Schwungmasse bestimmt gleichzeitig die Resonanzfrequenz des schwingenden Systems. Ergänzend sei angemerkt, dass die Steuerungseinrichtung 12 auch in diese Resonanz-/Schwungmasse 24 integriert sein könnte, um eine weitere Verkleinerung des Instrumentes 4 zu erreichen. Der an dem Instrument 4 vorgesehene Wandler 26 grenzt an die zuvor erwähnte Resonanz-/Schwungmasse 24 an. Bei dem Wandler 26 handelt es sich um einen piezoelektrischen Wandler 26, der in der vorliegenden Ausführungsform einen ähnlichen Aufbau wie der zuvor beschriebene piezoelektrische Transformator 14 hat. Auch der piezoelektrische Wandler 26 setzt sich aus mehreren übereinander geschichteten, ebenen Piezoelementen zusammen, wobei hier vier Piezoelemente 28, 30, 32, 34 vorgesehen sind, bei denen es sich um Quarz-Bauelemente handelt. Im Gegensatz zu dem Transformator 14 wird die über die Leitung 22 zur Verfügung gestellte elektrische Ausgangsspannung jedoch in eine mechanische Ultraschallschwingung jedes einzelnen Piezoelementes 28, 30, 32, 34 des Wandlers 26 umgewandelt, die auf die Resonanz-/Schwungmasse 24 und einen Werkzeughalter 36 übertragen wird.

Andere Ausführungsformen mit mehr oder auch mit weniger Piezoelementen und/oder Piezoelementen aus Piezokeramik statt Quarz sind denkbar.

An dem Werkzeughalter 36 ist ein Werkzeug 38 angeordnet, bei dem es sich um einen Zahnsteinmeißel zur Entfernung von Zahnstein handelt. Das Werkzeug 38 weist die klassische hakenförmige Gestalt eines Zahnsteinmeißels auf und ist austauschbar an dem Werkzeughalter 36 befestigt, so dass die Bedienungsperson das Werkzeug 38 entsprechend der jeweiligen Anwendung oder bei Verschleiß des Werkzeugs 38 austauschen und einen Austausch des Werkzeugs 38 bei einem Wechsel des Patienten durchführen kann. Die von dem Wandler 26 über den Werkzeughalter 36 auf das Werkzeug 38 übertragene mechanische Ultraschallschwingung führt zu einer Zerstörung des Zahnsteines, sobald das Werkzeug 38 mit diesem in Berührung kommt.

Zur Kühlung des Systems ist eine Kühlvorrichtung in Form einer Flüssigkeitskreislaufs 40 vorgesehen, der mittels nicht dargestellter Wärmetauscher die Abwärme des Systems einschließlich der Abwärme des Transformators aufnimmt und abtransportiert.

Die Resonanz-/Schwungmasse 24 mit allen Piezoelementen der Anregung und der Transformation, die Steuerungseinrichtung 12 sowie die Kühlung 40 sind in einem Gehäuse 42 untergebracht, an dessen Bearbeitungsseite noch eine Leuchte 44 vorgesehen ist, die an den Niederspannungsteil an die Steuerungseinrichtung 12 angeschlossen ist.

Fig. 2 zeigt die schematische Darstellung einer zweiten Ausführungsform der erfindungsgemäßen zahnärztlichen Vorrichtung 40 zur Behandlung mittels Ultraschall. Die zweite Ausführungsform entspricht weitgehend der ersten Ausführungsform, so dass im Folgenden lediglich die Unterschiede zu der ersten Ausführungsform dargestellt werden und im Übrigen auf die vorstehende Beschreibung verwiesen sein soll, wobei für gleichartige Komponenten die gleichen Bezugszeichen verwendet wurden.

Der wesentliche Unterschied zwischen der ersten Vorrichtung 2 und der zweiten Vorrichtung 2' besteht darin, dass die Energieversorgungseinrichtung 8' bei der zweiten Vorrichtung 2' in das Instrument 4' integriert ist. Auf diese Weise ist kein Kabel 10 zur Versorgung des Instrumentes 4' mehr erforderlich, was dessen Handhabung ungemein vereinfacht. Auch das periphere Gerät 6 kann vollständig entfallen. Bei der Energieversorgungseinrichtung 8' kann es sich um Energiespeicher, wie beispielsweise Batterien oder Akkumulatoren, handeln. Im letzteren Fall ist es sinnvoll, eine separate Ladeeinrichtung 40 vorzusehen, über die die Akkumulatoren wieder aufgeladen werden können.

## Patentansprüche

1. Zahnärztliche Vorrichtung zur Behandlung mittels Ultraschall mit einem Transformator (14) zur Umwandlung einer elektrischen Eingangsspannung in eine höhere elektrische Ausgangsspannung und einem Instrument (4, 4'), das einen Wandler (26) zur Umwandlung der elektrischen Ausgangsspannung in eine mechanische Ultraschallschwingung aufweist, wobei der Transformator (14) ein piezoelektrischer Transformator (14) ist, der unmittelbar an dem Instrument (4, 4') angeordnet ist, **dadurch gekennzeichnet, dass** der piezoelektrische Transformator (14) an einer durch den Wandler (26) in Schwingung versetzten Schwungmasse (24) angeordnet ist.

2. Zahnärztliche Vorrichtung nach Anspruch 1, **dadurch gekennzeichnet, dass** der piezoelektrische Transformator (14) im Bereich eines Schwingungsbauchs der Resonanz-/Schwungmasse (24) angeordnet ist.

3. Zahnärztliche Vorrichtung nach Anspruch 1, **dadurch gekennzeichnet, dass** der piezoelektrische Transformator (14) mechanisch entkoppelt an der Resonanz-/Schwungmasse (24) angeordnet ist.

4. Zahnärztliche Vorrichtung nach einem der Ansprüche 1 bis 3, **dadurch gekennzeichnet, dass** der Wandler (26) ein piezoelektrischer Wandler (26) ist.

5. Zahnärztliche Vorrichtung nach einem der Ansprüche 1 bis 4, **dadurch gekennzeichnet, dass** der piezoelektrische Transformator (14) und/oder der piezoelektrische Wandler (26) mehrere übereinander geschichtete, ebene Piezoelemente (18, 20, 28, 30, 32, 34) umfasst.

6. Zahnärztliche Vorrichtung nach einem der Ansprüche 1 bis 5, **dadurch gekennzeichnet, dass** eine Steuerungseinrichtung (12) zur Steuerung der Vorrichtung (2, 2') vorgesehen ist, wobei die Steuerungseinrichtung (12) in das Instrument (4, 4') integriert ist.

7. Zahnärztliche Vorrichtung nach einem der Ansprüche 1 bis 6, **dadurch gekennzeichnet, dass** in ein Gehäuse (42) des Instruments eine Energieversorgungseinrichtung (8') integriert ist.

8. Zahnärztliche Vorrichtung nach einem der Ansprüche 1 bis 7, **dadurch gekennzeichnet, dass** eine Lampe (44) am Niederspannungsteil des Transformators angeschlossen ist und in ein Gehäuse (42) des Instruments (4, 4') integriert ist.

9. Zahnärztliche Vorrichtung nach einem der Ansprüche 1 bis 8, **dadurch gekennzeichnet, dass** das Instrument (4, 4') mit einer Einrichtung zur Kühlung (40) ausgestattet ist und die Kühleinrichtung (40) so ausgelegt ist, dass der Transformator (14) durch die Kühleinrichtung (40) mitgekühlt wird.

## Claims

1. A dental device for applying supersonic treatment, comprising a transformer (14) for the transformation of an electrical input voltage to a higher electrical output voltage and an instrument (4, 4') including a transducer (26) for the conversion of said electrical output voltage to a mechanical ultrasonic vibration, said transformer (14) being a piezoelectric transformer (14) that is disposed directly against said instrument (4, 4'), **characterized in that** said piezoelectric transformer (14) is disposed adjacent to an oscillating weight (24) that is caused to vibrate by said transducer (26).

2. The dental device as defined in claim 1, **characterized in that** said piezoelectric transformer (14) is disposed in the region of an antinode of said resonance mass/oscillating weight (24).

3. The dental device as defined in claim 1, **characterized in that** said piezoelectric transformer (14) is disposed on said resonance mass/oscillating weight (24) mechanically decoupled therefrom.

4. The dental device as defined in any one of claims 1 to 3, **characterized in that** said transducer (26) is a piezoelectric transducer (26).

5. The dental device as defined in any one of claims 1 to 4, **characterized in that** said piezoelectric transformer (14) and/or said piezoelectric transducer (26) comprise a plurality of superposed planar piezo elements (18, 20, 28, 30, 32, 34).

6. The dental device as defined in any one of claims 1 to 5, **characterized in that** a control device (12) is provided for controlling said device (2, 2'), which control device (12) is integrated in said instrument (4, 4').

7. The dental device as defined in any one of claims 1 to 6, **characterized in that** energy supply means (8') for the instrument are integrated in a housing (42).

8. The dental device as defined in any one of claims 1 to 7, **characterized in that** a lamp (44) is connected to the low-voltage winding of said transformer and is integrated in a housing (42) of said instrument (4, 4').

9. The dental device as defined in any one of claims 1 to 8, **characterized in that** said instrument (4, 4') is equipped with cooling means (40) and said cooling means (40) are adapted such that said transformer (14) is also cooled by said cooling means (40).

## Revendications

1. Dispositif dentaire destiné au traitement par ultrasons comprenant un transformateur (14) pour convertir une tension d'entrée électrique en une tension de sortie électrique plus élevée et un instrument (4, 4') qui présente un transducteur (26) pour convertir la tension de sortie électrique en une vibration ultrasonore mécanique, sachant que le transformateur (14) est un transformateur piézoélectrique (14) qui est disposé directement sur l'instrument (4, 4'), **caractérisé en ce que** le transformateur piézoélectrique (14) est disposé sur une masse d'inertie (24) déplacée en vibration par le transducteur (26).

2. Dispositif dentaire selon la revendication 1, **caractérisé en ce que** le transformateur piézoélectrique (14) est disposé au niveau d'un ventre de vibrations de la masse d'inertie/de résonance (24).

3. Dispositif dentaire selon la revendication 1, **caractérisé en ce que** le transformateur piézoélectrique (14) est disposé sur la masse d'inertie/de résonance (24) en étant découplé mécaniquement.

4. Dispositif dentaire selon l'une des revendications 1 à 3, **caractérisé en ce que** le transducteur (26) est un transducteur piézoélectrique (26).

5. Dispositif dentaire selon l'une des revendications 1 à 4, **caractérisé en ce que** le transformateur piézoélectrique (14) et/ou le transducteur piézoélectrique (26) comprend/comprennent plusieurs éléments piézo (18, 20, 28, 30, 32, 34) plats mis en couche l'un sur l'autre.

6. Dispositif dentaire selon l'une des revendications 1 à 5, **caractérisé en ce qu'**un dispositif de commande (12) pour commander le dispositif (2, 2') est prévu, sachant que le dispositif de commande (12) est intégré dans l'instrument (4, 4').

7. Dispositif dentaire selon l'une des revendications 1 à 6, **caractérisé en ce qu'**un dispositif d'alimentation en énergie (8') est intégré dans un boitier (42) de l'instrument.

8. Dispositif dentaire selon l'une des revendications 1 à 7, **caractérisé en ce qu'**une lampe (44) est raccordée sur la partie basse tension du transformateur et est intégrée dans un boitier (42) de l'instrument (4, 4').

9. Dispositif dentaire selon l'une des revendications 1 à 8, **caractérisé en ce que** l'instrument (4, 4') est doté d'un dispositif de refroidissement (40) et que le dispositif de refroidissement (40) est ainsi conçu que le transformateur (14) est aussi refroidi par le dispositif de refroidissement (40).
